# EUROPEAN PATENT APPLICATION

(11) **EP 4 471 791 A1**
(43) Date of publication of application: **04.12.2024**
(21) Application number: 24177403.3
(22) Date of filing: 22.05.2024
(51) Int. Cl.: G16H 30/40, G16H 50/20, G06T 7/00

(54) **ARTIFICIAL INTELLIGENCE-BASED MEDICAL IMAGE ANALYSIS METHOD AND SYSTEM**

(30) Priority: 01.06.2023 KR 20230070631; 02.02.2024 KR 20240016863
(71) Applicant: Lunit Inc., Seoul, 06241 (KR)
(72) Inventor: JUNG, Haein, 06241 Seoul (KR); HA, Yuri, 06241 Seoul (KR)
(74) Representative: Germain Maureau

(57) **Abstract**

A device for analyzing an image includes: a memory; and a processor for executing instructions stored in the memory. The processor is configured to obtain an analysis result for an input medical image using a first artificial intelligence model and a second artificial intelligence model trained to analyze medical images with different tasks, and classify the input medical image into a normal case when the input medical image is determined to be normal by both the first artificial intelligence model and the second artificial intelligence model.

## Description

### BACKGROUND

### (a) Field

The present disclosure relates to an artificial intelligence-based medical image analysis technology.

### (b) Description of the Related Art

In recent years, with the rising integration of artificial intelligence (AI) technology in the healthcare, AI-based medical image analysis technologies, such as the Lunit INSIGHT solution, have been studied, where AI analyzes medical images and presents the results visually.

Radiologists may be assigned image reading tasks through a worklist, and perform image reading tasks of detecting abnormalities provided by medical image analysis and writing reports. Radiologists may sort worklists and change the image reading order, so that the readers may preferentially perform read tasks to urgent images or images in which abnormality is detected. However, worklist sorting only changes the order in which images already in the worklist are read, so the workload of the radiologists does not change, as the normal images still need to be read eventually. The proportion of normal images in the total images varies by country, hospital size, and clinical environment, but there is a significant proportion of normal images in which lesions are not generally detectable, so that a method to improve reading efficiency is demanded.

### SUMMARY

The present disclosure attempts to provide an artificial intelligence-based medical image analysis method and system.

The present disclosure attempts to provide an interface screen providing an artificial intelligence-based medical image analysis result.

The present disclosure attempts to provide a method of providing a worklist by utilizing an artificial intelligence-based medical image analysis result.

The present disclosure attempts to provide a method of generating a report by utilizing an artificial intelligence-based medical image analysis result.

An exemplary embodiment of the present disclosure provides a device for analyzing an image, the device including: a memory; and a processor for executing instructions stored in the memory. The processor is configured to obtain analysis results for an input medical image using a first artificial intelligence model and a second artificial intelligence model trained to analyze medical images with different tasks, and classify the input medical image into a normal case when the input medical image is determined to be normal by both the first artificial intelligence model and the second artificial intelligence model.

The first artificial intelligence model may be a normal filtering model trained to comprehensively detect abnormal findings in a medical image with more conservative criteria than the second artificial intelligence model, and the second artificial intelligence model may be a model trained to detect at least one specific lesion in a medical image.

The processor may be configured to determine whether the input medical image is normal based on a non-normal score obtained as an analysis result by the first artificial intelligence model and a normal filtering threshold.

The processor may be configured to change a current normal filtering threshold to a new value based on user input.

The processor may be configured to determine a new normal filtering threshold for normal filtering based on at least one of a type of medical institution performing the input medical image analysis, a normal case distribution or an abnormal case distribution of the medical institution for the non-normal score, and suggest the new normal filtering threshold to a user.

The processor may be configured to determine whether the input medical image is normal based on a per-lesion score obtained as an analysis result by the second artificial intelligence model.

The processor may be configured to classify the input medical image into a non-normal case when the input medical image is determined to be non-normal by the first artificial intelligence model, and the input medical image is determined to be normal or non-normal by the second artificial intelligence model.

The processor may be configured to generate a report for the input medical image classified into the normal case by using phrases preset for describing the normal case.

The processor may be configured to provide the analysis results for the input medical image as secondary capture (SC) in DICOM format, and the secondary capture may include a graphical indicator indicating that the input medical image is the normal case.

Another exemplary embodiment of the present disclosure provides a method of operating an image analysis device, the method including: obtaining analysis results for an input medical image using a first artificial intelligence model and a second artificial intelligence model trained to analyze medical images with different tasks; and classifying the input medical image into a normal case when the input medical image is determined to be normal by both the first artificial intelligence model and the second artificial intelligence model; and providing a final analysis result including the analysis results for the input medical image and normal case information indicating whether the input medical image is the normal case, to a designated device.

The first artificial intelligence model may be a normal filtering model trained to comprehensively detect abnormal findings in a medical image with more conservative criteria than the second artificial intelligence model, and the second artificial intelligence model may be a model trained to detect at least one specific lesion in a medical image.

The classifying of the input medical image into a normal case may include: determining whether the input medical image is normal based on a non-normal score obtained as an analysis result by the first artificial intelligence model and a normal filtering threshold, and determining whether the input medical image is normal based on a per-lesion score obtained as an analysis result by the second artificial intelligence model.

The method may further include changing a current normal filtering threshold to a new value based on user input.

The method may further include: determining a new normal filtering threshold for normal filtering based on at least one of a type of medical institution performing the input medical image analysis, a normal case distribution or an abnormal case distribution of the medical institution for the non-normal score; and suggesting the new normal filtering threshold to a user.

The method may further include classifying the input medical image into a non-normal case when the input medical image is determined to be non-normal by the first artificial intelligence model, and the input medical image is determined to be normal or non-normal by the second artificial intelligence model.

The method may further include generating a report for the input medical image classified into the normal case by using phrases preset for describing the normal case.

The final analysis result for the input medical image may be provided as secondary capture (SC) in DICOM format, and the secondary capture may include a graphical indicator indicating that the input medical image is the normal case.

Still another exemplary embodiment of the present disclosure provides a computer program stored in a computer-readable recording medium, the computer program including instructions to cause a processor executing the computer program to: display a worklist including a list of images for image reading work in association with an image storage device storing analysis results for medical images, and display medical images classified into a normal case in the worklist distinguishably based on whether the medical images are the normal case included in the analysis results for the medical images.

The computer program may further include instructions to cause the processor to: display a secondary capture (SC) image representing analysis results for a specific medical image when the specific medical image classified into the normal case is selected from the worklist. The secondary capture image may include a graphical indicator indicating that the particular medical image is the normal case.

The computer program may further include instructions to cause the processor to: provide a report for a medical image classified into the normal case. The report for the medical image classified into the normal case may be automatically generated by using phrases preset for describing the normal case.

According to the exemplary embodiments, medical images may be analyzed by using artificial intelligence models trained to analyze medical images with different tasks to increase accuracy and confidence in the analysis results.

According to the exemplary embodiments, by classifying normal medical images with high confidence among medical images and providing graphical indicators to recognize normal cases, the reading efficiency may be increased by reducing the reading time and workload, and consequently, the memory resources and computing resources of the medical image system for managing medical images waited for to be read may be reduced.

According to the exemplary embodiments, by classifying normal medical images with a high degree of confidence among medical images, a medical institution using the system may reduce reading costs.

According to the exemplary embodiments, by automatically generating a report based on the analysis results for the medical image, the reading efficiency may be increased by reducing the reading time and reading workload, and consequently, the memory resources and computing resources of the medical image system for managing medical images waited to be read may be reduced.

According to the exemplary embodiment, reading work for normal cases may be assigned to less skilled users or assigned to remote reading companies to efficiently run in-hospital reading work, thereby reducing the memory resources and computing resources of the medical image system for managing medical images waited to be read.

According to the exemplary embodiment, users may identify normal cases in a worklist that contain a list of images for image reading work and determine a work priority, and set a priority so that a non-normal case is read before a normal case.

According to the exemplary embodiment, a user may perform reading work by spending more time for reading non-normal cases than normal cases, and may reduce the time spent for reading normal cases through the SC image, the GSPS, and the like which includes an indication indicating that the medical image is the normal case.

### BRIEF DESCRIPTION OF THE DRAWINGS

FIG. 1 is a diagram illustrating a medical image system according to an exemplary embodiment.
FIG. 2 is a diagram illustrating a method of classifying a medical image into a normal case and an abnormal case according to an exemplary embodiment.
FIGS. 3 to 5 are diagrams illustrating examples of auxiliary images providing the analysis results according to an exemplary embodiment.
FIG. 6 is a diagram illustrating a method of setting a normal filtering threshold according to an exemplary embodiment.
FIG. 7 is a diagram illustrating an example of a worklist generated according to an exemplary embodiment.
FIG. 8 is a diagram illustrating an example of a report generated according to an exemplary embodiment.
FIG. 9 is a flowchart of an image analysis method according to an exemplary embodiment.
FIG. 10 is a flowchart of a method of setting a threshold for normal filtering according to an exemplary embodiment.
FIG. 11 is a flowchart of a method of providing an analysis result according to an exemplary embodiment.

### DETAILED DESCRIPTION OF THE EXEMPLARY EMBODIMENTS

Hereinafter, exemplary embodiments of the present disclosure will be described with reference to accompanying drawings so as to be easily understood by a person ordinary skilled in the art. The present disclosure may be variously implemented and is not limited to the following exemplary embodiments. In addition, in order to clearly explain the present description in the drawings, parts irrelevant to the description are omitted, and similar parts are denoted by similar reference numerals throughout the specification.

In addition, unless explicitly described to the contrary, the word "comprise", and variations such as "comprises" or "comprising", will be understood to imply the inclusion of stated elements but not the exclusion of any other elements. In addition, the terms "-er", "-or", and "module" described in the specification mean units for processing at least one function and operation, and may be implemented by hardware components or software components, and combinations thereof.

A device or a terminal of the present disclosure is a computing apparatus configured and connected so that at least one processor performs the operations of the present disclosure by executing instructions. A computer program may include instructions that cause a processor to execute the operations of the present disclosure and may be stored on a non-transitory computer readable storage medium. The computer program may be downloaded through a network or sold as a product.

Medical images of the present disclosure may be images of various parts of the body taken with various modalities, for example, the modalities may be x-rays, magnetic resonance imaging (MRI), ultrasound, computed tomography (CT), digital mammography (MMG), and digital breast tomosynthesis (DBT).

A user in the present disclosure is a healthcare professional, and may include, but is not limited to, a physician, nurse, clinical pathologist, sonographer, or medical imaging professional.

An artificial intelligence model (AI model) of the present disclosure is a machine learning model for learning at least one task, which may be implemented as a computer program executable by a processor. The task that an AI model learns may refer to the task desired to be solved with machine learning, or the task desired to be performed with machine learning. An AI model may be implemented as a computer program that runs on a computing device, and may be downloaded through a network, or sold as a product. Alternatively, an AI model may work with a variety of devices through a network.

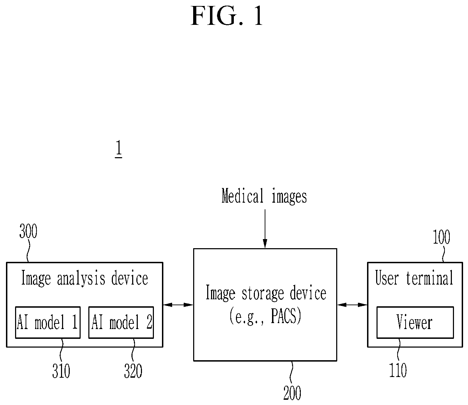

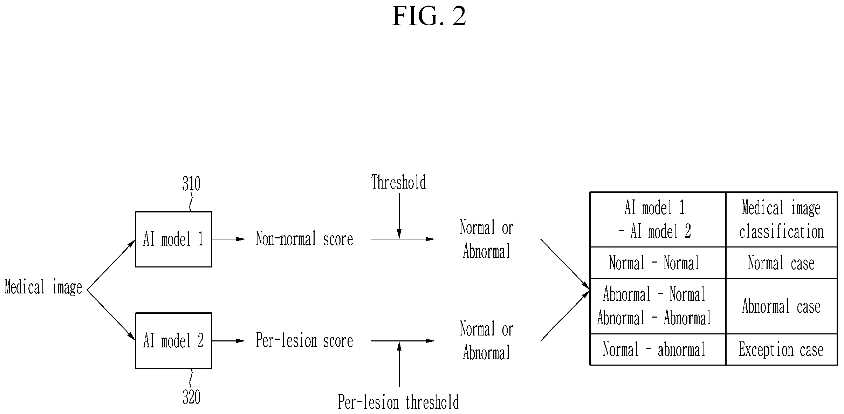

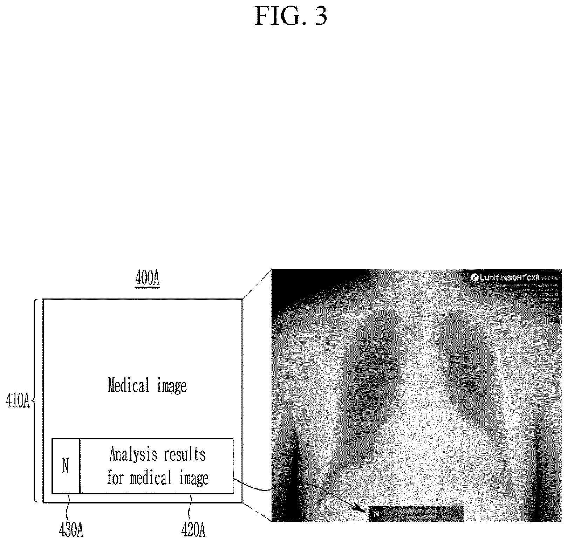

FIG. 1 is a diagram illustrating a medical image system according to an exemplary embodiment, FIG. 2 is a diagram illustrating a method of classifying a medical image into a normal case and an abnormal case according to an exemplary embodiment, and FIGS. 3 to 5 are diagrams illustrating examples of auxiliary images providing the analysis results according to an exemplary embodiment.

Referring to FIG. 1, a medical image system 1 may include at least one user terminal 100, an image storage device 200, and an image analysis device 300.

The user terminal 100 includes hardware and software that installs programs executed by a processor and provides a computing environment and network environment for performing the operations of the present disclosure. The user terminal 100 may be implemented in various types, such as, for example, a computing device in a workstation, or a mobile device. The user terminal 100 may include a viewer (simply referred to as "viewer") 110 that is associated with the image storage device 200 to display medical image-related data stored in the image storage device 200. The viewer 110 may be installed and executed on, for example, a computing device within a workstation, may be implemented to access the image storage device 200, and may display medical image-related data stored on the image storage device 200. The viewer 110 is a computer program stored on a computer-readable medium and includes instructions executed by a processor. The processor of the user terminal 100 may execute the instructions to perform the operations described herein.

The viewer 110 may display an analysis result for an image stored in the image storage device 200. The viewer 110 may provide a worklist that is organized in a tabular format and lists and shows a list of images to be read by the user along with key information. The viewer 110 may include a picture archiving and communication system (PACS) viewer. Here, the viewer 110 is a program designed to display medical images and/or image analysis results stored in the image storage device 200 and may support image reading operation associated with the worklist, but is not necessarily limited to a viewer for reading operation.

The image storage device 200 may store and manage taken medical images. The image storage device 200 may store and manage the analysis results for the medical images. The image storage device 200 may include a PACS database. The image storage device 200 may store data according to a specified data format. For example, the image storage device 200 may store medical images taken by medical imaging devices in accordance with the digital imaging and communications in medicine (DICOM) standard, and the results of analyzing the medical images, and may communicate with the user terminal 100 to provide data for reading the images. The image storage device 200 and the viewer 110 may be configured as a PACS system, and the image storage device 200 may be a PACS server/DB and the viewer 110 may be a PACS viewer.

The DICOM standard utilized for medical image storage is described herein as an example, but the medical image standard needs not be limited to DICOM.

The image storage device 200 may obtain analysis results for the medical image from the image analysis device 300. The analysis results for the medical image may include lesion information and various medical predictions. The analysis results for the medical image may be provided to assist a user in reading the image. The result of the image analysis may be provided in various formats, for example, Secondary Capture (SC) in DICOM format, or Grayscale Softcopy Presentation State (GSPS). The SC is the generation of a separate image (SC image) from the original medical image to display lesion information, which is provided separately from the original medical image and may be displayed in the PACS viewer. GSPS is the display of lesion information overlaid on top of the original medical image, and the overlaid lesion information may be turned on or off and displayed in the PACS viewer. The present disclosure will be generally described based on the SC as an example. The analysis results for the medical image may also be provided as a report in the form of a textual report. For example, the report may be a DICOM Basic Text Structured Report (SR). However, the form of providing the analysis results for the medical image is not limited thereto and may include results in various DICOM formats.

The medical images stored in the image storage device 200 may include images obtained by medical image devices of various modalities. The medical image may be x-ray images, magnetic resonance imaging (MRI) images, ultrasound images, computed tomography (CT) images, digital mammography (MMG) images, digital breast tomosynthesis (DBT) images, and the like. The present disclosure describes a chest x-ray image as an example of a medical image, but the medical image needs not be limited thereto, and the present disclosure may be adapted to any type of medical image.

The image analysis device 300 may analyze medical images using artificial intelligence (AI) models and store the analysis results in the image storage device 200.

The image analysis device 300 may have an AI model specialized for each type of medical image, and may select an AI model according to the type of input image to perform an analysis, such as detecting a lesion, suitable for the input image. The AI model is generated to make medical inferences from the input medical image, and the model structure, training data composition, training method, and medical inference target may be designed in various ways.

The image analysis device 300 may obtain the analysis results for medical images by using a plurality of AI models trained to analyze medical images with different tasks and generate a final analysis result for the medical images based on the results of the analysis. A report may be automatically generated based on the analysis results for medical images. The description assumes that the image analysis device 300 automatically generates the report, but the generation of the report may be performed by another device.

The number of AI models used by the image analysis device 300 is not necessarily limited to two, but in the description, it is assumed that AI model 1310 and AI model 2 320 are used. In this case, the use of AI model 1310 may be determined selectively. That is, the image analysis device 300 may analyze medical images by using both AI model 1 310 and AI model 2 320, or may analyze medical images by using only AI model 2 320, depending on the setting.

Referring to FIG. 2, AI model 1310 may be a model trained to comprehensively detect abnormal findings in medical images on a more conservative basis than AI model 2 320. AI model 1 310 may be trained to detect clinical abnormal findings, as well as lesions, such as nodules, pneumothorax, pleural fluid, sclerosis, cardiomegaly, atelectasis, emphysema, calcific degeneration, pulmonary fibrosis, mediastinal widening, pulmonary tuberculosis, and fractures. AI model 1310 may be trained to detect more types of lesions as abnormal findings than AI model 2 320. Here, clinical abnormal findings may include surgical marks, treatment marks, medical devices such as catheters, and the like, and may include information that a doctor needs to check for diagnosis or treatment.

AI model 1310 may be referred to as a normal filtering model or a comprehensive analysis model, in that AI model 1 310 comprehensively detects abnormal findings in medical images and classifies normal images that do not contain lesions as well as clinical abnormal findings that should be checked by a doctor.

AI model 1 310 may output a score associated with abnormal findings detected in the medical image, which may be referred to as a non-normal score. A non-normal score is a term used to distinguish a score associated with abnormal findings determined by AI model 1310 from an abnormality score, and a non-normal score may be interchangeably referred to as other terms. A non-normal score may be a value associated with a confidence level that AI model 1 310 is confident that abnormal findings exist in the medical image. The non-normal may be defined as a score with a value in a certain range (e.g., 0 to 100), or it may be defined as a probability value between 0 and 1. For example, when the non-normal score is defined with a probability value between 0 and 1, the normal score may be defined with a value obtained by subtracting the non-normal value from 1.

When the non-normal score predicted by AI model 1310 for the medical image is less than a threshold, the medical image may be classified as normal, otherwise, the medical image may be classified as abnormal. The threshold is a threshold for normal filtering, and the number of cases classified as normal may vary by the threshold. The threshold may be variable. The threshold may be adjusted by the user, may be adjusted to an optimal value by the image analysis device 300, or the optimal value may be suggested by the image analysis device 300 to the user.

AI model 2 320 may be a model trained to detect specific lesions in medical images. The lesions detectable in the chest x-ray image may include nodules, pneumothorax, pleural effusion, consolidation, cardiomegaly, atelectasis, pneumomediastinum, calcification, fibrosis, mediastinal widening, tuberculosis, acute bone fracture, and the like, as shown in Table 1.

**(Table 1)**

| Abbreviation | English lesion name |
|---|---|
| Ndl | Nodule |
| Ptx | Pneumothorax |
| PEf | Pleural effusion |
| Csn | Consolidation |
| Cm | Cardiomegaly |
| Atl | Atelectasis |
| Ppm | Pneumoperitoneum |
| Calc | Calcification |
| Fib | Fibrosis |
| MW | Mediastinal Widening |
| TB | Tuberculosis |
| Fx | Acute Bone fracture |

AI model 2 320 may output a score associated with the lesions detected in the medical image, which may be referred to as a per-lesion score. The per-lesion score may be a value associated with a confidence level that AI model 2 320 is confident that the lesion is present in the medical image.

Based on the per-lesion score predicted by AI model 2 320 for the medical image, the medical image may be classified as normal or abnormal. For example, when at least one lesion having the per-lesion score having a lesion score equal to or greater than a threshold is present among a plurality of detectable lesions, a medical image may be classified as abnormal. The threshold may be set on a lesion-by-lesion basis. Further, the per-lesion threshold may be adjusted by the user or adjusted to an optimal value by the image analysis device 300, or the image analysis device 300 may recommend an optimal value to the user. For example, when the user thinks that a result of the detection of a particular lesion is more important, a threshold for the particular lesion may be lowered.

When the predicted lesion score for the medical image is less than the threshold, an indication, such as "Low," may be output to indicate that the possibility that the corresponding lesion exists is low.

The abnormality score of the medical image may be determined based on the analysis result by AI model 2 320, for example, the highest lesion score among the per-lesion score may be determined as the abnormality score.

When the medical image is determined to be normal by both AI model 1310 and AI model 2 320, the image analysis device 300 may classify the medical image into a normal case. That is, the medical image may be classified into a normal case when no clinical abnormal findings, as well as no lesions, are detected. The image analysis device 300 may provide a score, such as a non-normal score or a normal score, indicating a confidence level that the medical image is a normal case.

The image analysis device 300 may classify the medical image into an abnormal case when the medical image is determined to be abnormal by at least one of AI model 1 310 and AI model 2 320. Here, an abnormal case refers to a medical image that is not obviously normal, and a medical image in which a lesion is not detected but clinical abnormal findings, such as surgical traces, are detected may be classified as an abnormal case.

On the other hand, when AI model 1 310 is trained to detect abnormal findings in the medical image with more conservative criteria than AI model 2 320, the possibility that the medical image determined to be abnormal by AI model 2 320 will be determined to be normal by AI model 1 310 is low. Accordingly, the image analysis device 300 may classify medical images determined to be normal by AI model 1 310 and determined to be abnormal by AI model 2 320 as an exception case. The exception case may be processed in various ways, and for example, the image analysis device 300 may be implemented to report the occurrence of an exception case to a designated device. The collected exception case may be utilized to improve AI model 1 310 or AI model 2 320.

The function of determining whether the medical image is normal by using the analysis result by AI model 1310 may be enabled based on user settings. When AI model 1 310 is disabled and only AI model 2 320 is used to analyze the medical image, it may be set to classify the medical image into a normal case or an abnormal case based on the result of the analysis by AI model 2 320. Alternatively, even when the result of the analysis by AI model 2 320 is normal, since the analysis by AI model 1310 has not been performed, it may be set to consider the result of the analysis by AI model 1 310 as abnormal and classify the medical image into an abnormal case.

The image analysis device 300 may generate a final analysis result for the medical image based on the analysis results by AI model 1 310 and AI model 2 320. The final analysis result may include the analysis result including the non-normal score and the like predicted by AI model 1310, the analysis result including a per-lesion score, abnormality score, normal status, lesion information, and the like predicted by AI model 2 320, normal case information indicating whether the input medical image is a normal case classified based on the analysis results of the two models, a non-normal score for the normal case, medical indicators, information indicating whether AI model 1 310 is used, and the like.

Based on the analysis result for the medical image, the image analysis device 300 may provide the analysis results including lesion information in the medical image in a format, such as Secondary Capture (SC) of DICOM, Grayscale Softcopy Presentation State (GSPS), Structured report (SR), or the like. The analysis results for the medical image may be stored in the image storage device 200 and displayed on the user terminal 100 that is associated with the image storage device 200. When the medical image is a DICOM image, metadata of the image may be stored in a public tag and a private tag. The Public tag records information about the medical image according to the file structure prescribed by the DICOM standard. The private tag may be freely used by medical device manufacturers to add information that is not included in the Public tag to the DICOM image, so that the analysis results for the medical image may be recorded in the Private tag. For example, the private tag may include the analysis results obtained by each of AI model 1 310 and AI model 2 320, a normal case classified based on the analysis results the analysis results for the two models, a non-normal score for the normal case, medical indicators, whether AI model 1 310 is used, and the like.

The analysis result for the medical image may be provided to represent a normal case. For example, the SC image or GSPS for a normal case may be provided in a different format from the SC image or GSPS for an abnormal case so that the user is capable of quickly identifying the normal case.

The medical images classified as normal cases may be filtered and excluded from the worklist and stored in a separate folder. The user may check only the images classified as normal cases through the corresponding folder. Alternatively, normal cases are not excluded from the worklist, but may be labeled in the worklist to indicate that the medical images are normal cases. The user may identify and process the normal cases in the worklist.

The viewer 110 executed on the user terminal 100 may provide the analysis results for the medical images. The viewer 110 may display various information contained in the results of the analysis, and to this end, the analysis results may be stored in a DICOM format, which is a data format that may be displayed by the viewer 110. The analysis results for medical images may be provided in SC, GSPS, SR, or other formats.

Referring to FIG. 3, the SC image 400A for a normal case may include an image area 410A in which the medical image is displayed, and an information area 420A in which the analysis results for the medical image are displayed. The information displayed in the information area 420A may be defined in various ways. The information area 420A may display a non-normal score or a normal score indicative of a confidence level for a normal case, or a level compared to a score or threshold for a specified medical indicator and/or lesion (e.g., "Low" indicating a level lower than a threshold). In a normal case, the information area 420A may be omitted.

The SC image 400A for a normal case may further display a graphical indicator 430A to indicate that the medical image is a normal case. The location and display method of the graphical indicator 430A may be defined in a variety of ways. For example, the graphical indicator may be represented by an "N" icon. "N" may mean no visible abnormality or normal.

For example, when the chest x-ray image is determined to be normal by both AI model 1310 and AI model 2 320, the chest x-ray image may be classified into a normal case. The SC image provided in a normal case may display information specified in the information area 420A (e.g., Abnormality Score: Low, TB analysis score: Low) and may display the graphical indicator 430A indicating that the chest x-ray image is a normal case. The information area 420A may display an abnormality score of the medical image, which may be labeled as "Low" to indicate that the medical image is the normal case and therefore is lower than the threshold. On the other hand, the lesion score for tuberculosis (TB) may be displayed separately from the abnormality score and may be labeled with a TB analysis score.

Even when the analysis results of the image is provided in the GSPS format, information indicating a normal case (e.g., "N") may be overlaid on the medical image.

The user may intuitively recognize that the medical images have been determined to be normal by the AI models by the graphical indicator indicative of a normal case.

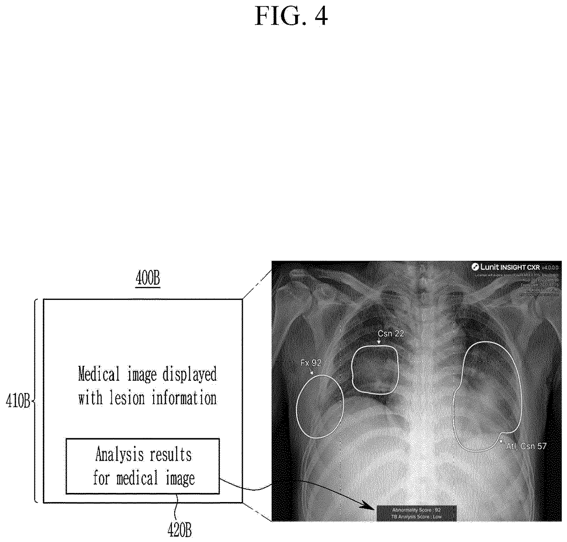

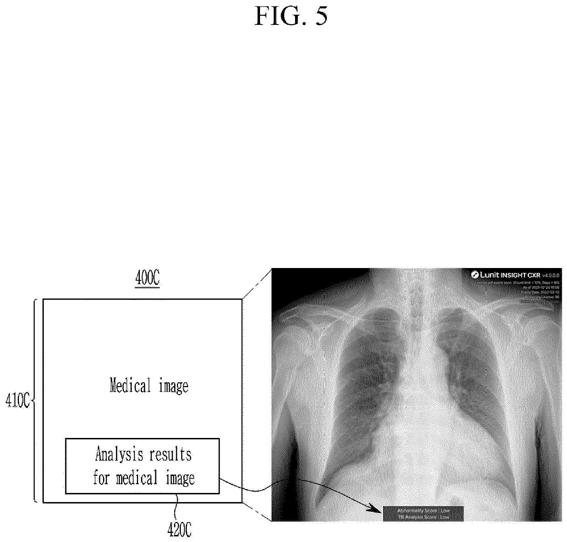

Referring to FIGS. 4 and 5, the SC images 400B and 400C for the abnormal cases may include image areas 410B and 410C where the medical images are displayed, and information areas 420B and 420C where the analysis results for the medical images are displayed. In this case, the SC images 400B and 400C for the abnormal case do not provide a graphical indicator to indicate that the medical image is a normal case. Instead, the SC images 400B and 400C may display a graphical indicator indicating that the medical image is an abnormal case.

When the medical image is determined to be abnormal by both AI model 1 310 and AI model 2 320, or when the medical image is determined to be abnormal by AI model 1310 and determined to be normal by AI model 2 320, the medical image may be classified as an abnormal case.

Referring to FIG. 4, when the medical image is determined to be abnormal by both AI model 1310 and AI model 2 320, the SC image 400B provided for the abnormal case may provide a medical image with visually displayed lesion information in the image region 410B, and the lesion information may include a location of the lesion region and a lesion score. For example, the imaging area 410B may display a lesion name and a per-lesion score, such as Fx 92, Csn 22, Atl, Csn 57, on the medical image, and may display the lesion area with a contour, heatmap, or the like.

The information area 420B may display specified information (e.g., Abnormality Score: 92, TB analysis score: Low) of the analysis results for the medical image. The information area 420B may display an abnormality score of the medical image, a per-lesion score, or a level compared to a per-lesion threshold (e.g., "Low" indicating a level less than the threshold). The abnormality score for the medical image may be the highest lesion score among the per-lesion scores. When the lesion score for a particular lesion is equal to or greater than the threshold, the lesion score may be displayed in the information area. When the lesion score for a particular lesion is less than the threshold, "Low" may be displayed to indicate a level less than the threshold.

Referring to FIG. 5, when the medical image is determined to be abnormal by AI model 1 310 and is determined to be normal by AI model 2 320, the SC image 400C provided to the abnormal case may not display the lesion information detected by AI model 2 320 in the image area 410C because it is judged to be normal by AI model 2 320, or may display the lesion information or clinical abnormal findings detected by AI model 1 310 in a specified way.

The information area 420C may display specified information, and for example, the information area 420C may display an indication indicative of a level of a specified lesion score lower than a threshold, an abnormality score, or a level of the abnormality score (e.g., "Low", "High").

Alternatively, whether to use AI model 1310 may be selectively determined, such that the SC image may display only the analysis result performed by AI model 2 320. When only the result of the analysis by AI model 2 320 is present, the medical image may be classified into an abnormal case because the medical image is not a normal case that has been determined to be normal by both models. When the analysis result for the medical image by AI model 2 320 is abnormal, the SC image for the medical image may be provided as shown in FIG. 4. When the analysis result for the medical image by AI model 2 320 is normal, the SC image of the medical image may be provided as shown in FIG. 5.

As such, the image analysis device 300 may analyze the medical image through AI model 1310 and AI model 2 320 to increase the accuracy and confidence of the results of the analysis. The image analysis device 300 may classify normal medical images with high confidence among medical images and provide graphical indicators for recognizing normal cases, thereby increasing reading efficiency by reducing reading time and reading workload, and consequently reducing memory resources and computing resources of the medical image system 1 for managing medical images waited to be read. Furthermore, the image analysis device 300 is capable of classifying normal medical images with high confidence among medical images, thereby enabling the medical institution using the image analysis device 300 to reduce reading costs.
the image analysis device 300 automatically generates a report based on the analysis results for the medical image by AI model 1 310 and AI model 2 320, thereby increasing the reading efficiency by reducing the reading time and the reading workload, and consequently reducing the memory resources and computing resources of the medical image system 1 for managing the medical image waited to be read.

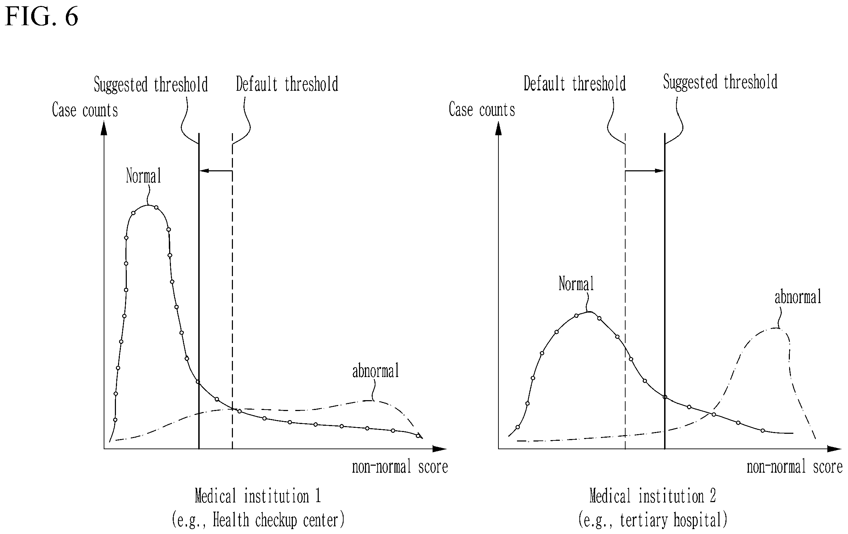

FIG. 6 is a diagram illustrating a method of setting a normal filtering threshold according to an exemplary embodiment.

Referring to FIG. 6, when a non-normal score predicted by AI model 1 310 for a medical image is less than a threshold, the medical image may be classified into normal; otherwise, the medical image may be classified into abnormal. The normal filtering threshold may be used identically across all medical institutions to classify medical images, but as the normal filtering thresholds vary the number of images determined to be normal by AI model 1 310, the number of images classified into normal cases may vary, which consequentially may affect the reading workload and reading efficiency.

Furthermore, referring to Table 2, different medical institutions have different patient characteristics, resulting in different proportions of normal cases, borderline cases, and abnormal cases. Accordingly, the normal filtering threshold may be adjusted to fit the distribution of cases in of the medical institutions

The normal filtering threshold may be adjusted by a change in a configuration value of the user or adjusted to an optimal value by the image analysis device 300, or the image analysis device 300 may recommend an optimal value to the user.

**(Table 2)**

| | Normal case (Completely Normal) | Borderline case (Clinically irrelevant) | Abnormal case (Clinically relevant) |
|---|---|---|---|
| Health checkup center | 40% | 30% | 30% |
| Secondary/Tertiary hospital | 15% | 20% | 65% |

When medical institutions are set to use AI model 1 310, the normal filtering threshold for determining normal images via AI model 1310 may be set to a default value. The image analysis device 300 may then statistically process the result data for the medical images analyzed by using AI model 1 310 and AI model 2 320 at each medical institution, and may change the normal filtering threshold or suggest an appropriate value based on the processed data. Here, the image analysis device 300 need not be limited to suggesting a single optimal value, but may suggest a range of adjustable normal filtering thresholds and receive a threshold within the range selected by the user.

The image analysis device 300 may provide the user with result data for the medical images in the form of graphs and/or dashboards, and may provide the currently set normal filtering threshold and a recommended normal filtering threshold together with the result data. Result data for medical images may include the number of cases, ratios, and case distributions for normal cases, borderline cases, and abnormal cases. The case distribution may be expressed as the number of corresponding cases for an abnormal score, and the definition of the normal case, the borderline case, and the abnormal case may vary. For example, the medical image determined as normal by the normal filtering threshold set in AI model 1310 may be determined as a normal case, or the medical image finally determined as normal by a reader may be determined as a normal case.

The image analysis device 300 may initiate an analysis of a medical image from a medical institution by using AI model 1 310. Further, a normal filtering threshold for a corresponding medical institution may be suggested based on data, such as normal case information (the number of normal cases, the ratio of normal cases, distribution of normal cases, and the like) for medical images from a medical institution analyzed by using the image analysis device 300 over a period of time, information about abnormal cases in which clinically relevant lesions were detected (the number of abnormal cases, the ratio of abnormal cases, distribution of abnormal cases, and the like), and the distribution of specialist findings for images acquired at the corresponding medical institution.

The image analysis device 300 may suggest a normal filtering threshold for each medical institution based on various information. For example, the image analysis device 300 may suggest a normal filtering threshold based on the distribution of normal cases, borderline cases, and abnormal cases of the medical institution. The image analysis device 300 may suggest normal filtering thresholds based on the type of medical institution.

For example, a case distribution of medical institution 1, such as a health checkup center, may have a large number of normal cases compared to abnormal cases, with normal cases skewed toward low non-normal scores. Furthermore, for the purposes of the health checkup center, the borderline cases should be suggested for further examination, so the image analysis device 300 may suggest a value lower than a default threshold as a normal filtering threshold corresponding to the non-normal score. In the case of the health checkup center, whether the medical image is normal is more conservatively determined by AI model 1 310 to achieve the checkup purpose, and since most cases are normal, lowering the normal filtering threshold does not cause a sharp increase in abnormal cases, and it is possible to classify a large number of medical images that are clearly normal by setting a low non-normal score as a threshold.

Based on the distribution of cases in medical institution 2, such as a tertiary hospital, there are many abnormal cases, and the borderline cases are mostly determined as clinically insignificantly lesions, so the image analysis device 300 may suggest a value higher than the default threshold as a normal filtering threshold corresponding to the non-normal score. In the case of medical images of patients visiting tertiary hospitals, the non-normal score is relatively high due to the high probability of detecting abnormal findings, such as lesions, so increasing the normal filtering threshold of AI model 1 310 does not cause a sharp increase in normal cases, and it is possible to classify medical images in which clinically important lesions are detected by setting a high non-normal score as a threshold.

As such, when the medical image is analyzed through AI model 1310, the medical image from medical institution 1 may be classified as non-normal even when the probability that an abnormal finding is present is lower than the default value, and the medical image from medical institution 2 may be classified as abnormal only when the probability that abnormal findings are present is higher than the default value. Through this, the ratio of normal images among the total images is different depending on countries, hospital sizes, and clinical settings, so that reading task may be optimized for each medical institution.

In one aspect, the user terminal 100 may provide an interface screen for adjusting a normal filtering threshold for AI model 1 310 and a per-lesion threshold for AI model 2 320. At this time, the interface screen may display the recommended threshold. The user may adjust at least one of the normal filtering threshold for AI model 1310 and the per-lesion threshold for AI model 2 320 via the interface screen, and the threshold adjusted by the user may be set in the image analysis device 300. Further, the user terminal 100 may provide the user with result data off the analysis of the medical images from each medical institution by the image analysis device 300 for a period of time in the form of graphs and/or dashboards, and the user may recognize characteristics of the medical institution based on the result data to set a definition of normal for each lesion. For example, the user may set a lesion score of 30 or less to be determined as normal for Calcification, set a lesion score of 25 or less to be determined as normal for Fibrosis, and set a medical device among clinical abnormal findings to be determined as normal.

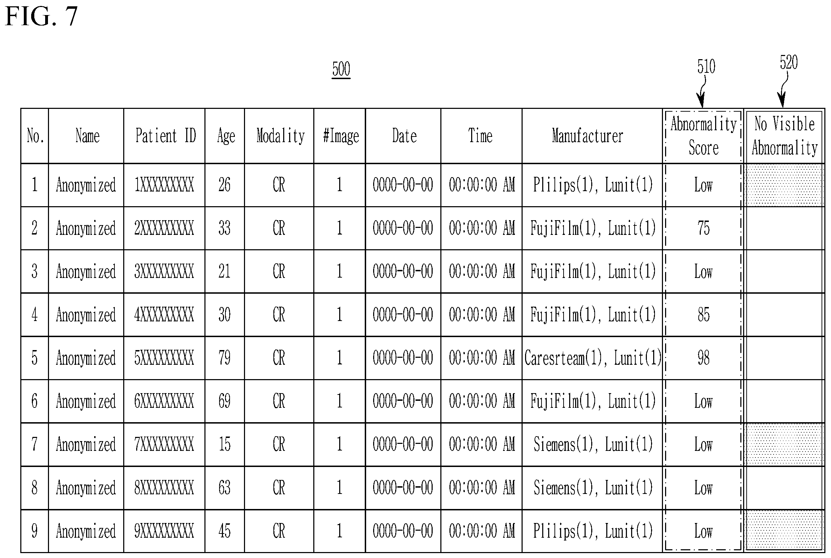

FIG. 7 is a diagram illustrating an example of a worklist generated according to an exemplary embodiment.

Referring to FIG. 7, the viewer 110 executed on the user terminal 100 may provide a worklist 500 in association with the image storage device 200. The worklist 500 presents a list of images to be read by a user in a tabular format with key information. The worklist does not necessarily need to be included in the viewer 110 and may be installed as a separate program. The information displayed in the worklist may be provided by the viewer 110 or the image storage device 200, which may be described as being provided by the viewer 110 for ease of description.

The viewer 110 may display the analysis results performed by the image analysis device 300 on the medical images in designated columns 510 and 520 of the worklist 500 in association with the image storage device 200. The analysis results displayed in the worklist may be varied, for example, an abnormality score for each image may be listed in the column 510 and a normal case may be listed in "No Visible Abnormality" column 520. For example, the viewer 110 may distinguish the data on the normal case included in the private tag of the medical image with a color chip or flagging and display the data in the work list. The viewer 110 may display the images included in the worklist in a distinct manner based on a priority determined by a result of a comparative analysis. Further, the viewer 110 may sort and display a list of images in the worklist according to the priority determined by the result of the comparative analysis.

When a particular medical image is determined to be normal by both AI model 1 310 and AI model 2 320, the worklist 500 may indicate (e.g., flag or highlight) that the particular medical image is a normal case in the "No Visible Abnormality" column 520, and may provide a function for sorting or extracting normal cases for normal case reading. This allows users (e.g., radiologists) to identify normal case and determine the priority of workload and to set priorities to read abnormal cases before normal cases. In addition, the user may spend more time to read abnormal cases than normal cases. The SC or GSPS of a normal case includes an indication that the medical image is a normal case, so the user may spend less time to read normal cases.

The worklist 500 may display the "No Visible Abnormality" column 520 when the function of determining whether a medical image is normal by using the analysis result of AI model 1 310 is enabled, and may hide the "No Visible Abnormality" column 520 when the function is disabled.

Alternatively, the worklist 500 may not display medical images of normal cases that are determined to be normal by both AI model 1 310 and AI model 2 320, or may provide the function to collect and view only normal cases by storing the normal cases in a separate folder. Alternatively, the normal cases may not be assigned to the worklist for reading, or when the report is automatically generated and stored, the worklist may display an indicator indicating that the report for the images of the normal cases has been automatically generated. The user may recognize that the report has been generated that requires confirmation from the user through the corresponding indicator. In other words, the medical image system 1 may provide a reading work procedure in which a user does not read a medical image of a normal case, and the report for the normal case may be automatically generated based on the analysis result for the medical image and stored in the image storage device 200. The image analysis device 300 may generate the report for the normal case and store the report in a designated folder of the image storage device 200. Regarding the normal case, the image analysis device 300 may generate the report based on the analysis results for the medical image, which may reduce the time required for a user to create the report. The automatically generated report may be modified by a designated user, or may be finalized and stored. Alternatively, the image storage device 200 or a separate device may generate the report based on the analysis results for the medical image.

Furthermore, the medical image system 1 may allocate reading work for normal cases to less skilled users or assign reading work for normal cases to remote reading companies to efficiently operate reading work in hospitals, thereby improving the quality of medical services provided to patients.

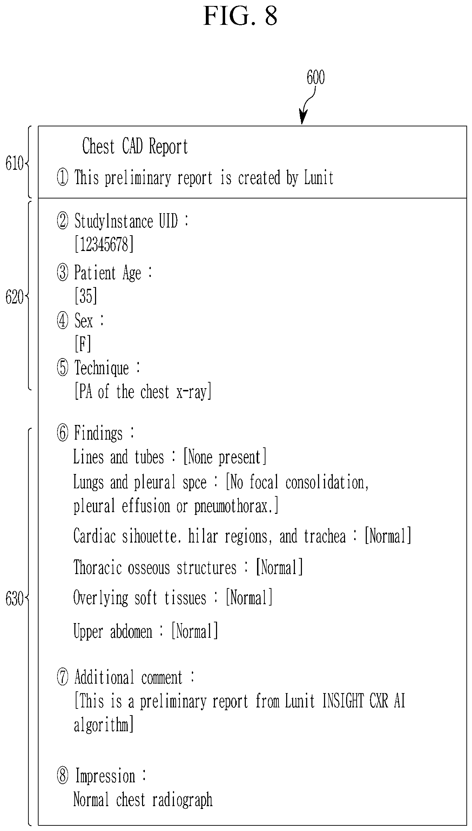

FIG. 8 is a diagram illustrating an example of a report generated according to an exemplary embodiment.

Referring to FIG. 8, the image analysis device 300 or a designated device may generate a report based on the analysis results for the medical image. For example, a report 600 may be generated for a normal case that is determined to be normal by both AI model 1 310 and AI model 2 320.

The report 600 may be structured, for example, as a name region 610, a patient and image information region 620, and an analysis region 630.

The name region 610 may include a name of a report (e.g., Chest CAD Report) and a description of the report, such as a description indicating that the report was automatically generated by an AI rather than a human (e.g., ① This preliminary report is created by Lunit.).

The patient and image information region 620 may include items, such as a medical image identifier (e.g., ② StudyInstanceUID: [...]), Patient Age (e.g., ③ Patient Age: [35]), Patient Sex (e.g., ④ Sex: [F]), and medical image information (e.g., ⑤ Technique: [PA of the chest x-ray]). These items may be set by the user in the settings page. The value listed in each item in the patient and image information region 620 may be extracted from the DICOM tag of the medical image as a corresponding value.

The analysis region 630 may include items, such as findings, additional comment, and impression. Similar to the items included in the patient and image information region 620, the items included in the analysis region 630 may be set by the user on the settings page. For example, the findings phrase for a chest x-ray image (e.g., ⑥ Findings: Lines and tubes: [None present], Lungs and pleural spce: [No focal consolidation, pleural effusion or pneumothorax.], Cardiac silhouette, hilar regions, and trachea: [Normal], Thoracic osseous structures: [Normal], Overlying soft tissues: [Normal], Upper abdomen: [Normal], etc.) may also be set by the user on a setting page. In a normal case, all findings may be described as normal. The comment phrase described in the additional comment (e.g., ⑦ Additional comment: [This is a preliminary report from Lunit INSIGHT CXR AI algorithm.]) may be set by the user on the settings page, and when the user hasn't entered any additional comments, the additional comments area (⑦) may not be displayed. In a normal case, the comprehensive opinion may state that the medical image is normal (e.g., ⑧ Impression: Normal chest radiograph).

In this way, the report for the normal case may be automatically generated in the format preset by the user. By automatically generating the report for the normal case, users (radiologists) may spend less time reading and writing the report for the normal image. On the other hand, in order to automate the generation of the report for the normal image, it is necessary to accurately classify normal images. The image analysis device 300 may increase the confidence of automatically generated report by classifying the medical image determined to be normal by both AI model 1310 and AI model 2 320 as the normal case.

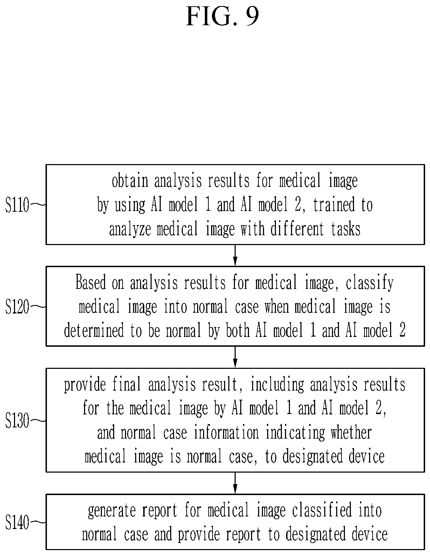

FIG. 9 is a flowchart of an image analysis method according to an exemplary embodiment.

Referring to FIG. 9, the image analysis device 300 obtains analysis results for the medical image by using AI model 1310 and AI model 2 320, which are trained to analyze the medical image with different tasks (S 1 10). AI model 1310 is a normal filtering model trained to comprehensively detect abnormal findings in medical images with more conservative criteria than AI model 2 320, and may detect lesions and clinical abnormal findings as abnormal findings. AI model 2 320 may be a model trained to detect specific lesions in medical images. The image analysis device 300 may obtain the analysis result including a non-normal score or the like predicted by AI model 1 310, and the analysis result including a per-lesion score, abnormality score, whether the medical image is normal, lesion information, or the like predicted by AI model 2 320. Meanwhile, the image analysis device 300 may analyze the medical image using AI model 1310 and AI model 2 320, or may analyze the medical image by using only AI model 2 320, depending on the setting.

Based on the analysis results for the medical image, the image analysis device 300 classifies the medical image into a normal case when the medical image is determined to be normal by both AI model 1310 and AI model 2 320 (S 120). The image analysis device 300 may determine that the medical image is determined to be normal by AI model 1310 when the abnormal score obtained as the analysis result by AI model 1 310 is below a normal filtering threshold. In this case, the normal filtering threshold for determining the medical image as normal or non-normal based on the non-normal score may be variable. The image analysis device 300 may determine whether the medical image is normal based on a per-lesion score obtained as the analysis result by AI model 2 320, or an abnormality score determined from the per-lesion score. The per-lesion threshold for determining the medical image as normal or non-normal based on the per-lesion score may be variable. The image analysis device 300 may change the normal filtering threshold, the per-lesion threshold, or the like according to user input. The image analysis device 300 may suggest a new normal filtering threshold to the user.

The image analysis device 300 provides a final analysis result, including the analysis results for the medical image by AI model 1310 and AI model 2 320, and normal case information indicating whether the medical image is a normal case, to the designated device (S130). The final analysis result for the medical image may be generated in DICOM format and provided to the image storage device 200. The final analysis result for the medical image may be stored in the image storage device 200, for example, a PACS server/DB, and displayed via the viewer 110 on the user terminal 100 that is in association with the image storage device 200.

The image analysis device 300 generates a report for the medical image classified into the normal case and provides the report to a designated device (S140). The image analysis device 300 may generate a report structured to include patient and image information extracted from the DICOM tag of the medical image, a set of preset normal finding phrases for the normal case, and a comprehensive opinion that the medical image is normal.

The medical image may be treated differently in the medical image system 1 depending on whether the medical image is the normal cases. An SC image or GSPS of the normal case may include a graphical indicator indicating that the medical image is a normal case. When the medical image of the normal case is assigned to a worklist, an indication (flag) indicating that the medical image is the normal case may be added. Alternatively, the medical image of the normal case may not be assigned to a worklist for reading, or may not appear in the worklist. Alternatively, the medical image of the normal case may be stored in a separate folder and made available to the user for viewing in a collection of the normal cases. Alternatively, when the report for the medical image of the normal case is automatically generated and stored, the worklist may display an indicator to indicate that the report for the corresponding medical image is automatically generated. Through the corresponding indicator, the user is capable of recognizing that the report has been automatically generated that requires confirmation from the user.

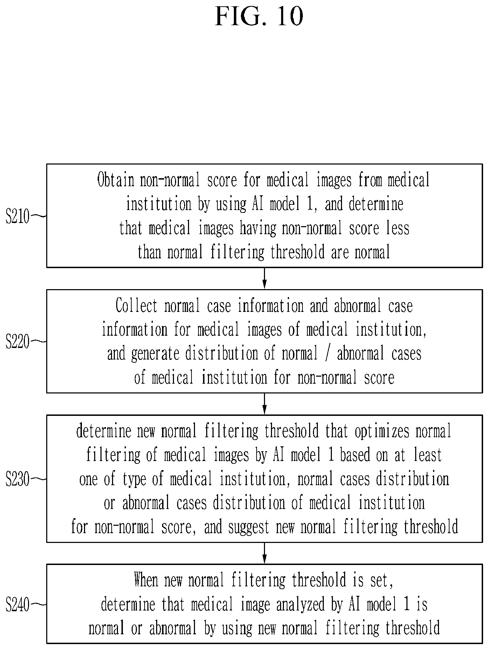

FIG. 10 is a flowchart of a method of setting a threshold for normal filtering according to an exemplary embodiment.

Referring to FIG. 10, the image analysis device 300 obtains a non-normal score for medical images from a medical institution by using AI model 1 310, and determines that medical images having the non-normal score less than a normal filtering threshold are normal (S210). AI model 1 310 is a normal filtering model trained to comprehensively detect abnormal findings in medical images with conservative criteria, and may detect lesions and clinical abnormal findings as abnormal findings. Further, the image analysis device 300 may analyze the medical images by using AI model 2 320, which is trained to detect specific lesions in the medical images, and classify the medical images that are determined to be normal by both AI model 1310 and AI model 2 320 as normal cases.

The image analysis device 300 collects normal case information and abnormal case information for medical images of a medical institution, and generates a distribution of normal/abnormal cases of the medical institution for the non-normal score (S220). The normal case information includes the number of normal cases, a ratio of normal cases, a distribution of normal cases, and the like, and the abnormal case information is information about cases in which clinically significantly lesions are detected, and may include the number of abnormal cases, a ratio of abnormal cases, a distribution of abnormal cases, and the like. The medical images of the normal cases and the abnormal cases are mapped with the non-normal score determined by AI model 1 310, and the image analysis device 300 may generate a graph indicating the number of normal and abnormal cases per abnormal score. The image analysis device 300 may provide the generated graph.

The image analysis device 300 determines a new normal filtering threshold that optimizes normal filtering of medical images by AI model 1310 based on at least one of the type of medical institution, the normal case distribution or the abnormal case distribution of the medical institution for the non-normal score, and suggest the new normal filtering threshold (S230). The logic for optimizing the normal filtering may be designed to vary depending on the optimization condition. The image analysis device 300 may provide the distribution of normal cases or the distribution of abnormal cases of the medical institution for the non-normal score and/or the proposed new normal filtering threshold or threshold range to a user via the viewer 110. The user may view the distribution of the cases of the medical institution and/or the proposed new normal filtering threshold or threshold range, and set the new normal filtering threshold in a setting window provided in the form of a user interface.

When the new normal filtering threshold is set, the image analysis device 300 determines that a medical image analyzed by AI model 1 310 is normal or abnormal by using the new normal filtering threshold (S240).

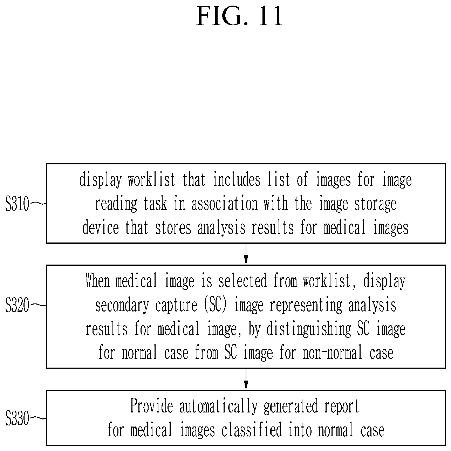

FIG. 11 is a flowchart of a method of providing an analysis result according to an exemplary embodiment.

Referring to FIG. 11, the viewer 110 displays a worklist that includes a list of images for an image reading task in association with the image storage device 200 that stores the analysis results for medical images (S310). The viewer 110 displays the analysis results of each image analyzed by the image analysis device 300 in a designated column of the worklist, and the analysis results may include an abnormality score, whether the medical image is a normal case, and the like. The worklist may provide the function of not-displaying or hiding the medical images of the normal case that are determined to be normal by both AI model 1310 and AI model 2 320. Alternatively, the normal case may not be assigned to the worklist for reading. Alternatively, when a report for the normal case is automatically generated and stored, a distinct marker may be provided on the worklist. When the report for the normal case is automatically generated and stored, an indicator may be displayed on the worklist to indicate that a report has been generated that requires confirmation from the user.

When a medical image is selected from the worklist, the viewer 110 displays a secondary capture (SC) image representing the analysis results for the medical image, by distinguishing an SC image for a normal case from an SC image for a non-normal case (S320). The viewer 110 may display the taken image of the medical image and the SC image in DICOM format. The SC image for the normal case may be provided in a different format from the SC image for the non-normal case to allow the user to quickly identify the normal case. For example, the SC image for the normal case may display a graphical indicator to indicate that the medical image is a normal case.

The viewer 110 provides the automatically generated report for the medical images classified into the normal case (S330). That is, the user does not need to manually write the report for the medical image classified as the normal case, but rather may check the report automatically generated by using preset normal finding phrases for the normal case and modify the report when necessary.

The viewer 110 is implemented as a computer program stored on a computer-readable recording medium and includes instructions executable by a processor. The computer program may include instructions that cause the processor executing the computer program, in association with the image storage device 200, to display a worklist including a list of images for an image reading operation and, when a medical image is selected in the worklist, to display the analysis results for the medical image.

The terminal or devices 100, 200, and 300 configuring the medical image system 1 of the present disclosure may include one or more processors, a memory for loading a computer program executed by the processor, a storage device for storing the computer program and various data, and a communication interface. In addition, the terminal or devices 100, 200, and 300 may further include various other components. A processor is a device that controls the operation of a server and may be various forms of processor that processes instructions contained in a computer program, and may include, for example, at least one of a Central Processing Unit (CPU), a Micro Processor Unit (MPU), a Micro Controller Unit (MCU), a Graphic Processing Unit (GPU), or any other form of processor well known in the art of the present disclosure. The memory stores various data, commands, and/or information. The memory may be implemented to store instructions such that the instructions described to execute the operations of the present disclosure are processed by the processor. The memory may be, for example, Read Only Memory (ROM) and Random Access memory (RAM). The storage device may store computer programs and other data on a non-transitory basis. The storage device may include a non-volatile memory, such as a Read Only Memory (ROM), an Erasable Programmable ROM (EPROM), an Electrically Erasable Programmable ROM (EEPROM), a flash memory, or the like, a hard disk, a removable disk, or any other form of computer-readable recording medium well known in the art to which the present disclosure belongs. The communication interface may be a wired/wireless communication module supporting wired/wireless communication. The computer program includes instructions executable by a processor and is stored on a non-transitory computer readable storage medium, and the instructions cause the processor to perform the operations of the present disclosure. Upon acquiring a target image of the patient, the image analysis device 300 may analyze the target image to obtain a analysis result including lesion information.

The exemplary embodiments of the present disclosure described above are not only implemented through the apparatus and method, but may also be implemented through programs that realize functions corresponding to the configurations of the exemplary embodiment of the present disclosure, or through recording media on which the programs are recorded.

Although an exemplary embodiment of the present disclosure has been described in detail, the scope of the present disclosure is not limited by the exemplary embodiment. Various changes and modifications using the basic concept of the present disclosure defined in the accompanying claims by those skilled in the art shall be construed to belong to the scope of the present disclosure.

## Claims

1. A device for analyzing an image, the device comprising:
a memory; and
a processor for executing instructions stored in the memory,
wherein the processor is configured to:
obtain analysis results for an input medical image using a first artificial intelligence model and a second artificial intelligence model trained to analyze medical images with different tasks, and
classify the input medical image into a normal case when the input medical image is determined to be normal by both the first artificial intelligence model and the second artificial intelligence model.

2. The device of claim 1, wherein
the first artificial intelligence model is a normal filtering model trained to comprehensively detect abnormal findings in a medical image with more conservative criteria than the second artificial intelligence model, and
the second artificial intelligence model is a model trained to detect at least one specific lesion in a medical image.

3. The device of claim 1, wherein the processor is configured to
determine whether the input medical image is normal based on a non-normal score obtained as an analysis result by the first artificial intelligence model and a normal filtering threshold, and
determine whether the input medical image is normal based on a per-lesion score obtained as an analysis result by the second artificial intelligence model.

4. The device of claim 3, wherein the processor is configured to change a current normal filtering threshold to a new value based on user input.

5. The device of claim 4, wherein the processor is configured to
determine a new normal filtering threshold for normal filtering based on at least one of a type of medical institution performing the input medical image analysis, a normal case distribution or an abnormal case distribution of the medical institution for the non-normal score, and
suggest the new normal filtering threshold to a user.

6. The device of claim 1, wherein the processor is configured to
classify the input medical image into a non-normal case when the input medical image is determined to be non-normal by the first artificial intelligence model, and the input medical image is determined to be normal or non-normal by the second artificial intelligence model.

7. The device of claim 1, wherein the processor is configured to
generate a report for the input medical image classified into the normal case, by using phrases preset for describing the normal case.

8. The device of claim 1, wherein the processor is configured to
provide the analysis results for the input medical image as secondary capture (SC) in DICOM format, and
the secondary capture includes a graphical indicator indicating that the input medical image is the normal case.

9. A method of operating an image analysis device, the method comprising:
obtaining analysis results for an input medical image using a first artificial intelligence model and a second artificial intelligence model trained to analyze medical images with different tasks;
classifying the input medical image into a normal case when the input medical image is determined to be normal by both the first artificial intelligence model and the second artificial intelligence model; and
providing a final analysis result including the analysis results for the input medical image and normal case information indicating whether the input medical image is the normal case, to a designated device.

10. The method of claim 9, wherein
the first artificial intelligence model is a normal filtering model trained to comprehensively detect abnormal findings in a medical image with more conservative criteria than the second artificial intelligence model, and
the second artificial intelligence model is a model trained to detect at least one specific lesion in a medical image.

11. The method of claim 9, wherein the classifying the input medical image into a normal case includes:
determining whether the input medical image is normal based on a non-normal score obtained as an analysis result by the first artificial intelligence model and a normal filtering threshold; and
determining whether the input medical image is normal based on a per-lesion score obtained as an analysis result by the second artificial intelligence model.

12. The method of claim 11, further comprising:
determining a new normal filtering threshold for normal filtering based on at least one of a type of medical institution performing the input medical image analysis, a normal case distribution or an abnormal case distribution of the medical institution for the non-normal score;
suggesting the new normal filtering threshold to a user; and
changing a current normal filtering threshold to the new normal filtering threshold based on user input.

13. The method of claim 9, further comprising:
classifying the input medical image into a non-normal case when the input medical image is determined to be non-normal by the first artificial intelligence model, and the input medical image is determined to be normal or non-normal by the second artificial intelligence model.

14. The method of claim 9, further comprising:
generating a report for the input medical image classified into the normal case, by using phrases preset for describing the normal case.

15. The method of claim 9, wherein
the final analysis result for the input medical image is provided as secondary capture (SC) in DICOM format, and
the secondary capture includes a graphical indicator indicating that the input medical image is the normal case.
